(19) Europäisches Patentamt
European Patent Office
Office européen des brevets

(11) **EP 3 090 781 A1**

(12) **EUROPEAN PATENT APPLICATION**

(43) Date of publication:
**09.11.2016 Bulletin 2016/45**

(21) Application number: **16001045.0**

(22) Date of filing: **09.05.2016**

(51) Int Cl.:
*A61Q 19/08* (2006.01)        *A61K 8/35* (2006.01)
*A61K 8/63* (2006.01)        *A61K 8/49* (2006.01)
*A61K 8/97* (2006.01)        *A61K 8/99* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **08.05.2015 EP 15001406**

(71) Applicant: **Kaufmann, Susanne
6870 Bezau (AT)**

(72) Inventor: **Kaufmann, Susanne
6870 Bezau (AT)**

(74) Representative: **Sattler de Sousa e Brito, Clara
ZSP Patentanwälte PartG mbB
Radlkoferstrasse 2
81373 München (DE)**

(54) **ANTI-AGE COMPOSITION WITH PLANT EXTRACTS**

(57)    In a first aspect the present invention provides a composition for the delay of at least one sign of skin ageing comprising a component A comprising a vitamin A like agent, ubiquinone, ectoin and/or plant phytosterols, a proteasome activity enhancer, anti-irritant and anti-inflammatory agent and a component B comprising a plant extract from *Cassia angustifolia.* In a second aspect the present invention provides the use of a composition for the treatment of and/or prevention of at least one sign of skin ageing, wherein the at least one sign of skin ageing is present on skin of the face, body or scalp of the subject.

EP 3 090 781 A1

**Description**

The present invention relates to a composition for the delay of at least one sign of skin ageing, which combat the effects of skin aging and/or provide anti-aging benefits comprising a component A comprising a vitamin A like agent; ubiquinone, ectoin and/or plant phytosterols, a proteasome activity enhancer, anti-irritant and anti-inflammatory agents and a component B comprising a plant extract from *Cassia angustifolia*.

The present invention further relates to the use of a composition according to claims 1 to 14 for the treatment of and/or prevention of at least one sign of skin ageing, wherein the at least one sign of skin ageing is present on skin of the face, body or scalp of the subject.

**Background of the invention**

The skin is subject to aging. It may be chronological or due to extrinsic factors. Both of them contribute to a loss of homeostasis and lead to physiological dysfunctions. The most upper layer of skin, the epidermis, is naturally more exposed to environment and cellular components are particularly sensitive to the induced damages. Aging is a multifactorial phenomenon. The aging of the skin is mainly the result of one's genetic predisposition (known as chronological aging) and one's physiological reaction to environmental stresses (known as actinic aging). Actinic aging seems to be skin specific and is defined as the effect of the external environment on the skin's biological response. The skin response to actinic aging, which may be caused by sun and pollution exposure, as well as smoking, is typically associated with a lack of normal hydration, apparition of telangiectasia (spider veins), sagging of the skin, and the appearance of fine lines and wrinkles. In addition, temporary or even lasting changes to the skin can occur with age, such as hormonal associated acne, greasy or dry skin, keratosis, rosacea, light sensitivity or inflammatory erythema. Moreover, many people are concerned with the degree of pigmentation of their skin and may wish to reduce darkening, or even lighten their 'natural' skin color. The process of keratinocyte differentiation and desquamation are essential for both intact barrier function and for healthy looking skin. By naturally aging, these functions decrease resulting in impaired barrier function, which leads to loss of moisture and ultimately to dry skin, but also increased susceptibility of the skin to external insults such as infection and photo damage. Sun-exposure of the skin causes thickening of the epidermis. This could be a result of senescent keratinocytes and melanocytes surviving for a prolonged time, possibly due to defective desquamation. Stimulating keratinocytes to maintain the balance of proliferating and differentiating keratinocytes, improves both barrier function and the appearance of the skin, thereby providing healthy younger looking skin. Mechanical methods can be used to stimulate desquamation. However, these methods commonly also have a proteolytic and/or keratinolytic activity, resulting in irritation of the skin. Thus, there is a need for compounds that stimulate desquamation without having the disadvantage of damaging other aspects of the skin. A further feature of aging skin is impaired or slowed wound healing. Wound healing is a complex process involving migration, proliferation, differentiation and infiltration of several different cell types and the production of numerous growth factors, cytokines and tissue restering molecules resulting in the synthesis of new tissue and wound closure. Retinoic acid is the gold standard anti-aging ingredient, but is not without uncomfortable side effects and regulatory restrictions on its use. Alternative retinoid-like actives have similar, but fewer, restrictions. A side effect of topical retinoid use is skin irritation. Irritated skin is characterized by redness, dryness and flaking at the treated site. At the histological level, a perivascular accumulation of mononuclear cells is observed, with neutrophils and monocytes scattered throughout the dermis and occasional micro-abscesses in the dermis or epidermis. Irritation is a major cause of non-compliance among retinoid users. In addition, excessive irritation may counteract the beneficial effects of topical anti-age compositions including retinoic acid derivative for example. Other signs of aging are the development of fine lines, lines and wrinkles, loss of skin elasticity and diminished skin regeneration capacity.

Thus, there remains a need for topical compositions which address the desire to look younger and reduce fine lines and wrinkles, increase cell elasticity and cell self renewal, protect from free radicals and active the skins own regeneration capacity but avoid dryness, irritation, inflammation, redness and other undesired effects. Furthermore, such compositions should be capable of increasing the desquamation and hydration of the skin, skin softness, and water holding capacity of the stratum corneum.

**Summary of the invention**

In a first aspect the present invention provides a composition for the delay of at least one sign of skin ageing comprising a component A comprising a vitamin A like agent, ubiquinone, ectoin and/or plant phytosterols, a proteasome activity enhancer, an anti-irritant and anti-inflammatory agent and a component B comprising a plant extract from *Cassia angustifolia*. In a second aspect the present invention provides the use of a composition for the treatment of and/or prevention of at least one sign of skin ageing, wherein the at least one sign of skin ageing is present on skin of the face, body or scalp of a subject.

**List of Figures**

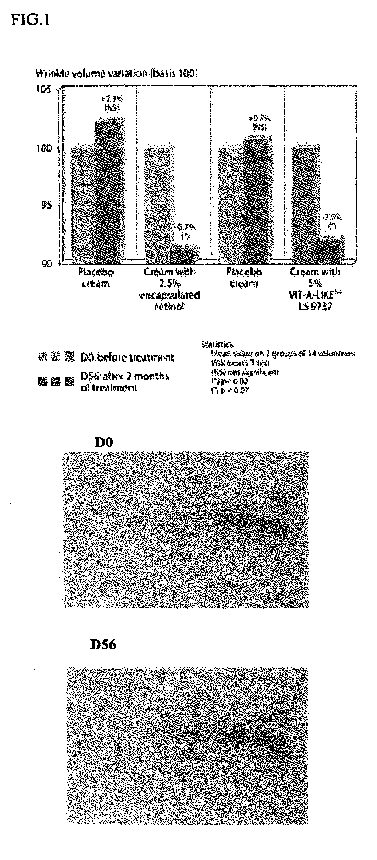

**Figure 1: VitaminA like agent (Vit A like PW LS 9898)**
A 5% Vit A like cream has shown an anti-wrinkle efficacy on the crow's foot area, which is as good as the benchmark (cream with 2,5% encapsulated retinol). Thanks to its cellular renewal activity, VIT-A-LIKE resurfaced, refined and youthfully radiant. Macrophotograph of the crow's foot area before and after 2 months of treatment with 5% vitamin A like agent cream.

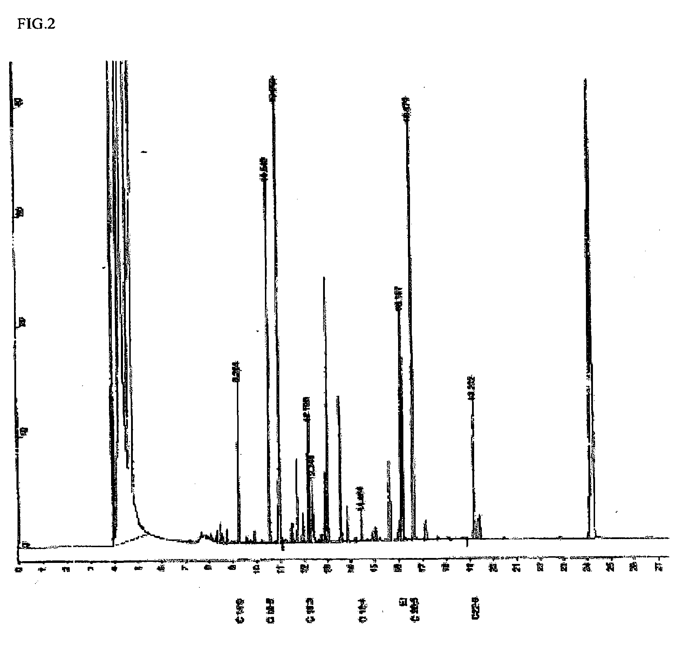

**Figure 2: Molecular Profile of *Phaeodactylum tricornutum extract***
The *Phaeodactylum tricornutum* extract presents a specific molecular profile. *Phaeodactylum tricornutum* contains a high content of polyunsaturated fatty acid (PUFAs), especially eicosapentaenoic acid. It presents a unique composition, very rich in specific fatty acids:

- C16:1
- C20:5, Eicosapentaenoic acid (EPA), an $\omega 3$ polyunsaturated fatty acid
- C22:6, DocosaHexaenoic Acid (DHA), an $\omega 3$ polyunsaturated fatty acid.

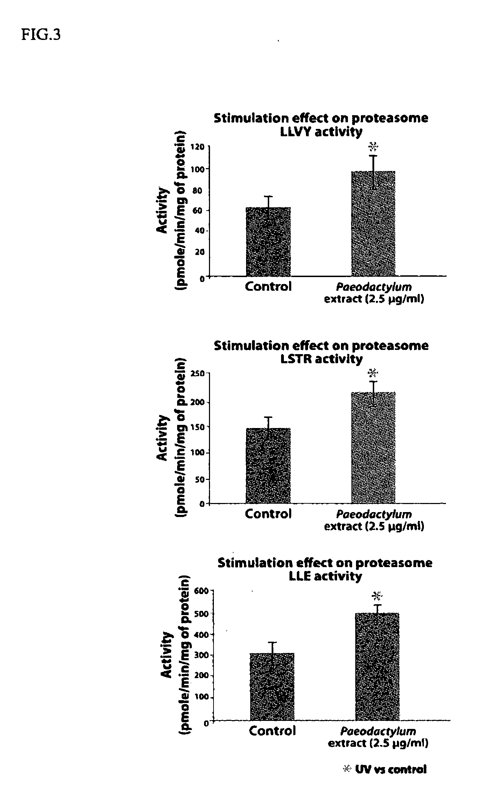

**Figure 3: Stimulation of proteasome activities *in vitro***

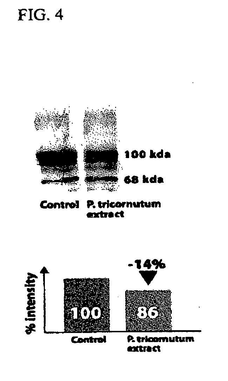

**Figure 4: Decrease of oxidized protein level during chronological aging *in vitro***

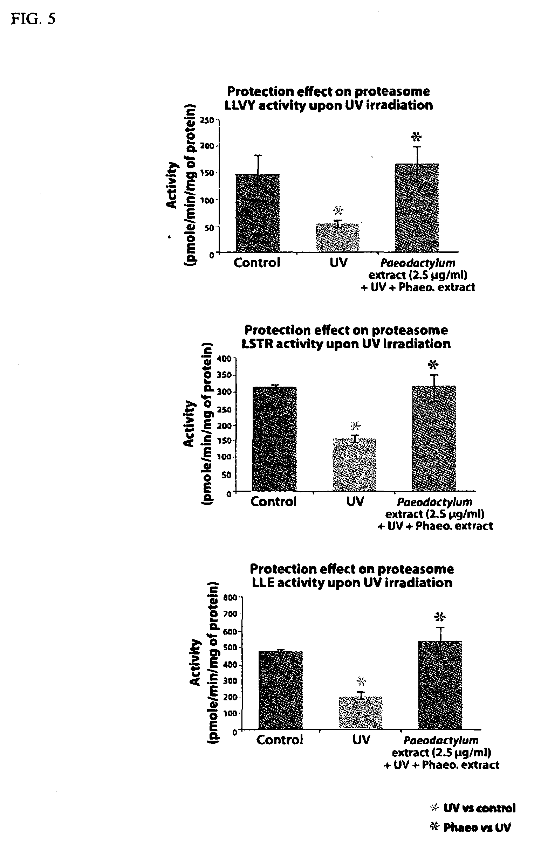

**Figure 5: Protection effect on proteasome activity upon UV irradiation**

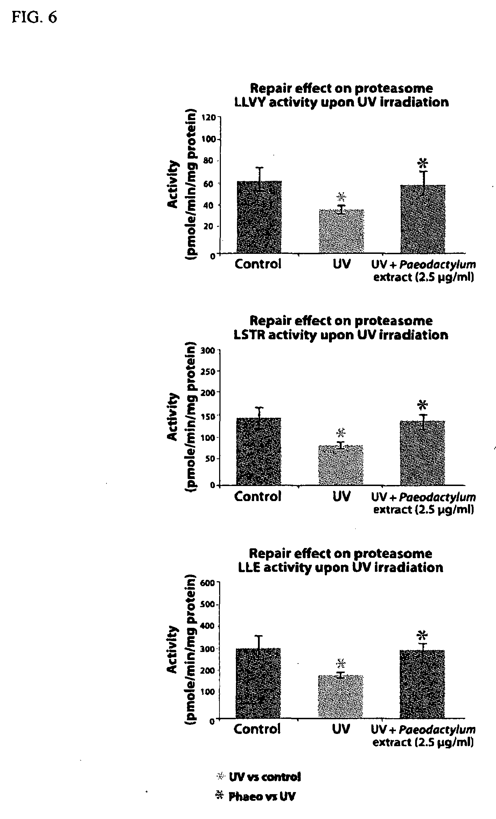

**Figure 6: Repair effect on proteasome activity upon UV irradiation**

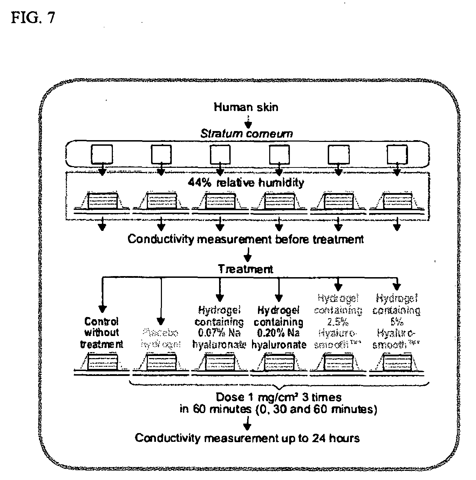

**Figure 7: Protocol for evaluation of moisturizing activity**

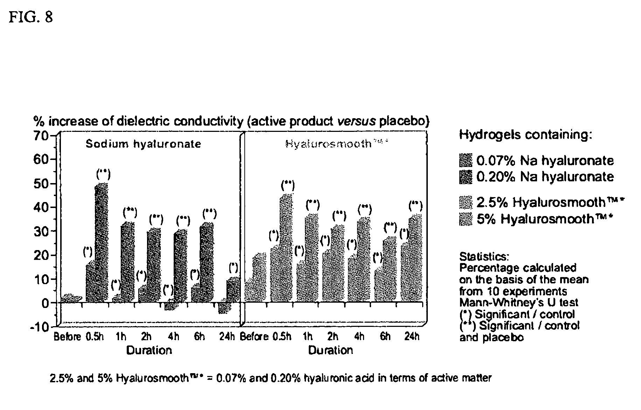

**Figure 8: Moisturizing activity evaluated by measurement of dielectric conductivity on the human horny layer *in vitro* at 44% relative humidity.**

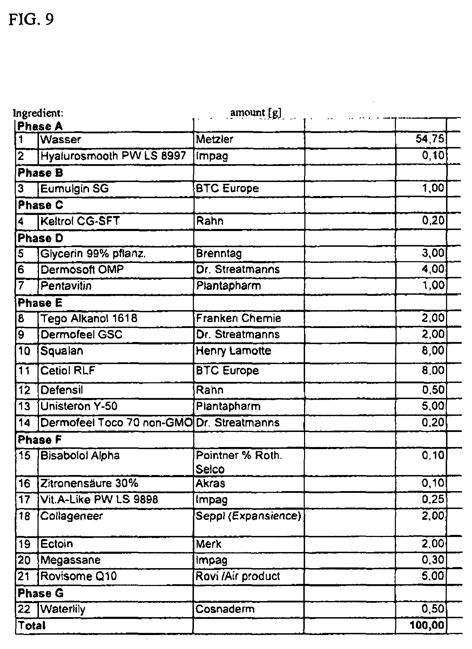

**Figure 9: Recipe of the composition of the present invention**

**Figure 10: Cutometer Parameters for measurement of biomechanical properties (elasticity, firmness)**

**Figure 11a: Skin hydration: experimental data (delta values)**
Evaluated are changes in the hydration values in the treated area in comparison to the changes in the untreated area. An increase in the measurement value corresponds to an increase in skin hydration. The absolute changes by area and time point are displayed. After both 14 and 28 days of treatment, a statistically significant ($p<0.05$) increase in skin hydration was observed in the product treated test area as compared to the changes in the untreated area.The test product was found to increase skin hydration; after 28 days of treatment a positive effect could be detected in 100% of the study participants.

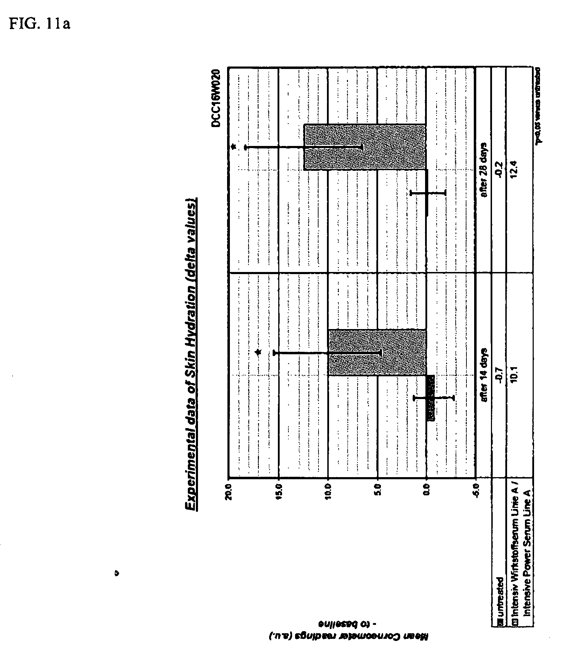

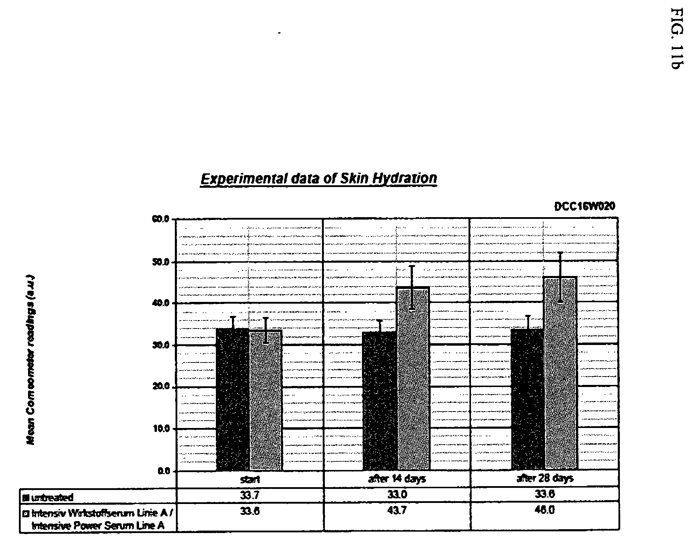

**Figure 11b: Skin hydration: experimental data**
The test product was found to statistically significantly increase skin hydration. After 28 days of treatment, a mean increase by 38% was observed and a positive effect of the test product was detected in 100% of the volunteers.

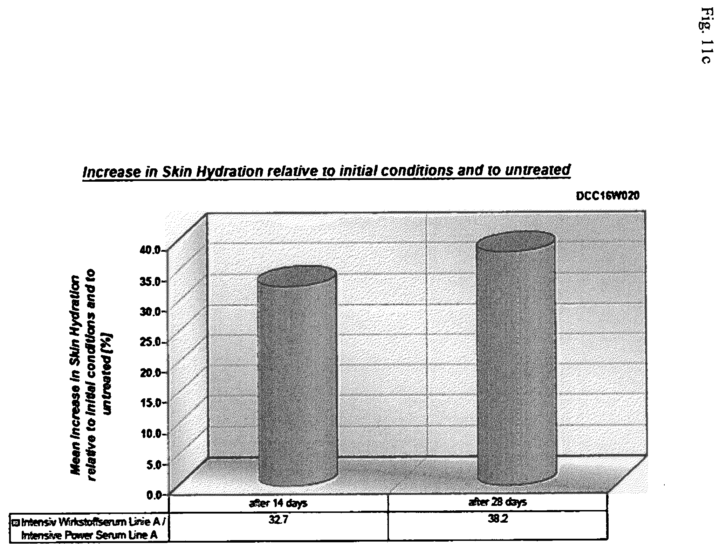

**Figure 11c: Skin hydration: experimental data (delta values): increase in skin hydration in %**
The respective changes in skin hydration as percentages relative to the initial condition and with consideration of the changes in the untreated area are summarized.

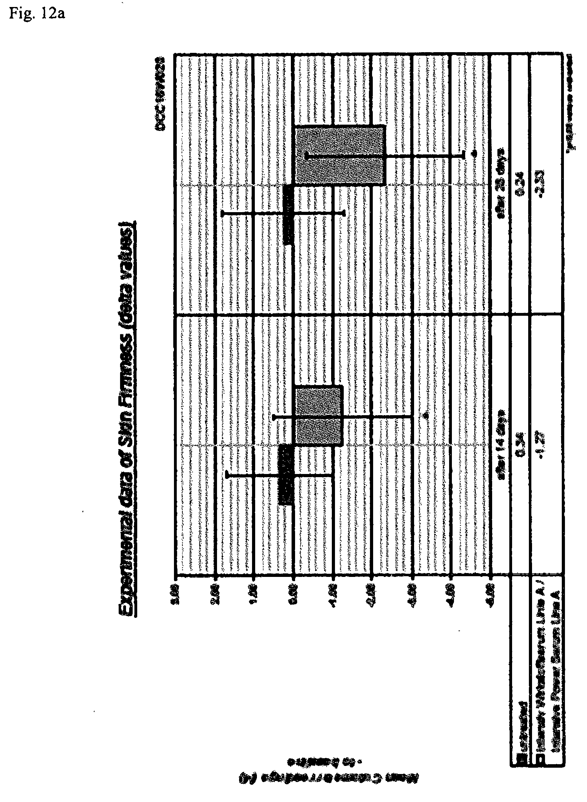

**Figure 12a: Skin firmness experimental data (delta values)**
In assessing skin firmness, evaluated are changes in the parameter F4 in the treated area in comparison to the changes in the untreated area. The absolute changes by area and time point are shown below in figure 12a. A decrease in F4 corresponds to an increase in skin firmness.

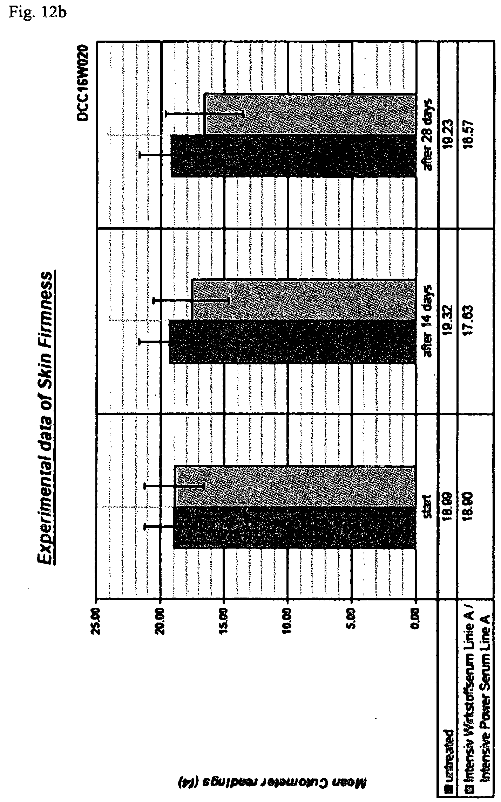

**Figure 12b: Skin firmness experimental data**

The test product was found not only to increase skin firmness, but at the same time to also increase skin elasticity. Overall, it induced a change in the biomechanical properties of the skin towards the firm-elastic optimum. The test product was found to statistically significantly enhance the biomechanical properties of the skin towards the firm-elastic optimum. After 28 days of treatment, a mean increase by 14% firmness was observed and a positive effect of the test product was detected in 95% of the volunteers.

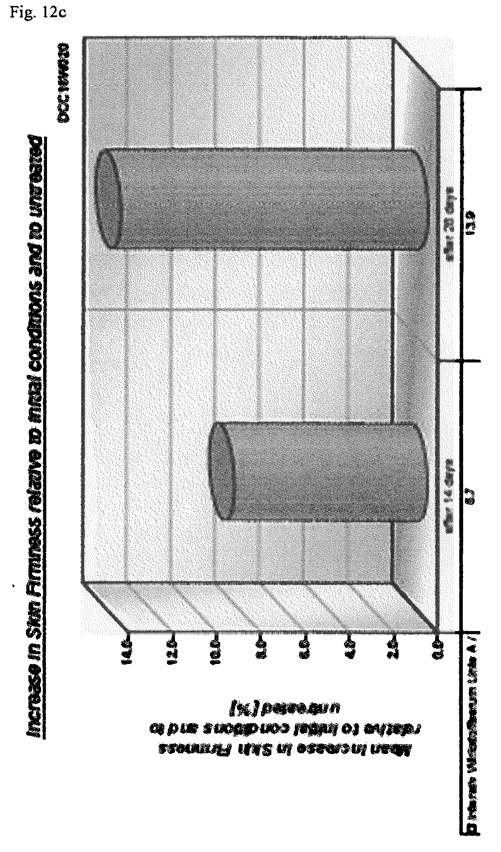

**Figure 12c: Skin firmness: experimental data (delta values): increase in skin firmness in %**
After both 14 and 28 days of treatment, a statistically significant ($p < 0.05$) decrease in F4 was observed in the product treated test area as compared to the changes in the untreated area. The test product was found to statistically significantly increase skin firmness; after 28 days of treatment a positive effect could be detected in 95% of the study participants. The respective percentage changes as compared to the initial condition and with regard of the changes in the untreated area are shown in figure 12c.

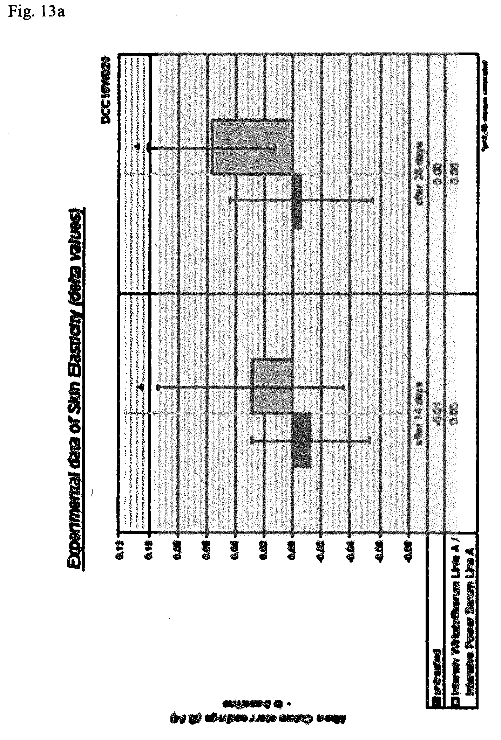

**Figure 13a: Skin elasticity: experimental data (delta values): increase in skin elasticity in**
In assessing skin elasticity, evaluated are the changes in the fraction F3 divided by F4 in the treated area in comparison to the changes in the untreated area. The absolute changes by area and time point are shown in figure 13a. An increase in F3/F4 corresponds to an increase in skin elasticity.

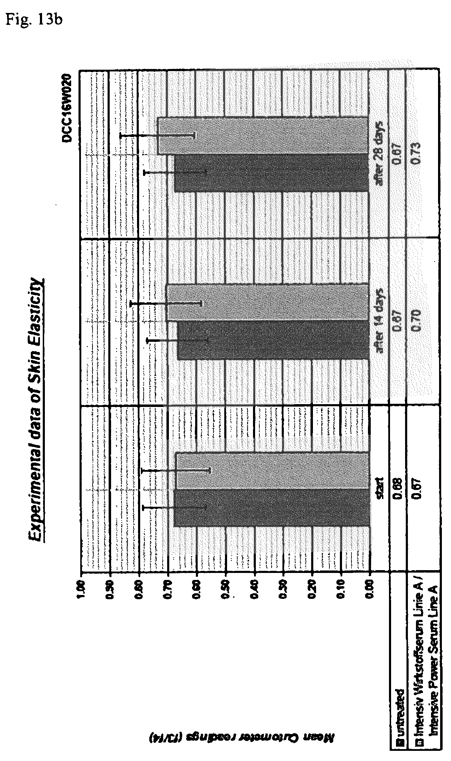

**Figure 13b: Skin elasticity: experimental data**
The test product was found not only to increase skin firmness, but at the same time to also increase skin elasticity. Overall, it induced a change in the biomechanical properties of the skin towards the firm-elastic optimum. The test product was found to statistically significantly enhance the biomechanical properties of the skin towards the firm-elastic optimum. After 28 days of treatment, a mean increase by 9% elasticity was observed and a positive effect of the test product was detected in 95% of the volunteers.

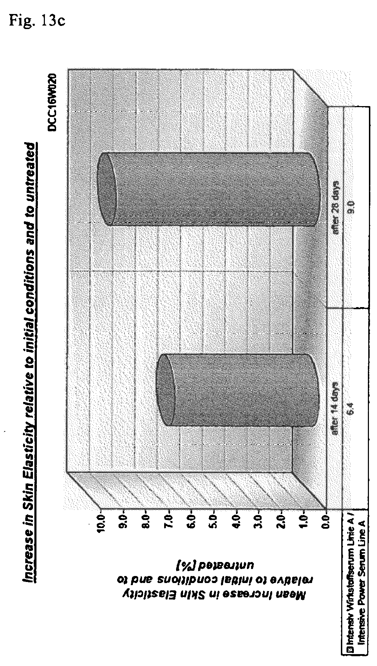

**Figure 13c: Skin elasticity: experimental data (delta values): increase in skin elasticity in %**
After both 14 and 28 days of treatment, a statistically significant ($p < 0.05$) increase in F3/F4 was observed in the product treated test area as compared to the changes in the untreated area. The test product was found to statistically significantly increase skin elasticity; after 28 days of treatment a positive effect could be detected in 95% of the study participants. The respective percentage changes as compared to the initial condition and with regard of the changes in the untreated area are shown in figure 13c.

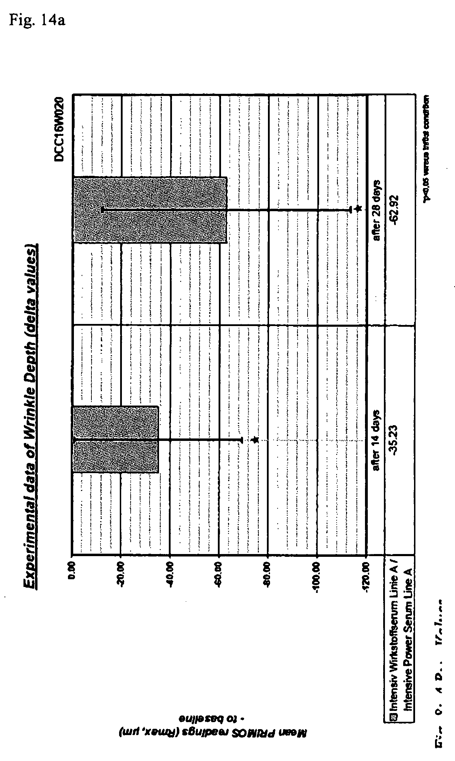

**Figure 14a: Wrinkle depth: Experimental data (delta values)**
Evaluated is the parameter $R_{Max}$ in comparison to the initial condition in the same respective test area. The absolute changes by time point are shown below in figure 14a. A decrease in $R_{Max}$ corresponds to a decrease in wrinkle depth.

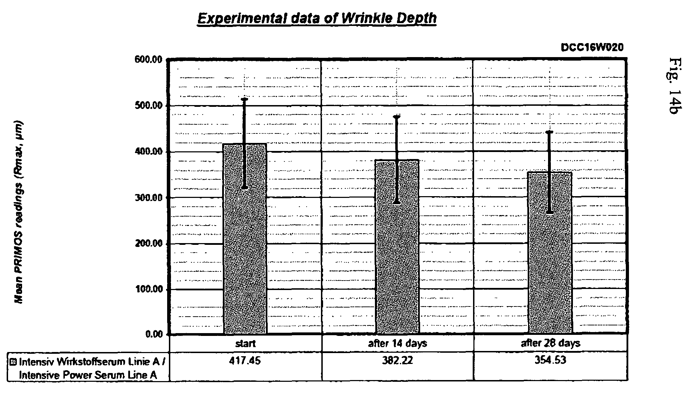

**Figure 14b: Wrinkle depth: Experimental data: Mean PRIMOS readings**
The test product was found to statistically significantly decrease wrinkle depth. After 28 days of treatment, a mean decrease by 15% was observed and a positive effect of the test product was detected in 95% of the volunteers.

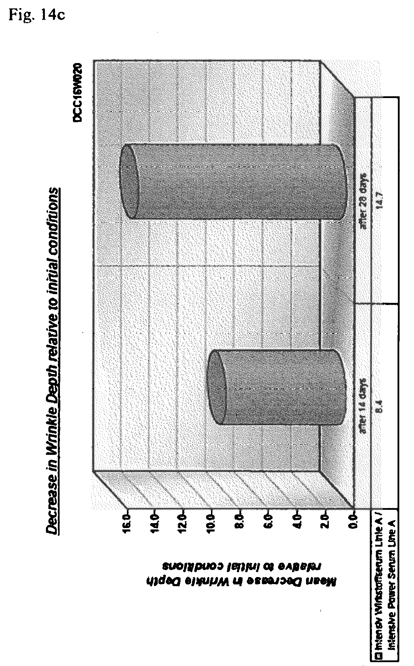

**Figure 14c: Wrinkle depth: Experimental data: Decrease in wrinkle depth in %**
After both 14 and 28 days of treatment, a statistically significant ($p < 0.05$) decrease in $R_{Max}$ was observed in the product treated condition as compared to the initial condition. The test product was found to statistically significantly decrease wrinkle depth; after 28 days of treatment a positive effect could be detected in 95% of the study participants. The respective percentage changes as compared to the initial condition are shown in figure 14c.

**Detailed Description of the Invention**

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention, which will be limited only by the appended claims. Unless defined otherwise, all technical and scientific terms used herein have the same meanings as commonly understood by one of ordinary skill in the art.

Several documents are cited throughout the text of this specification. Each of the documents cited herein (including all patents, patent applications, scientific publications, manufacturer's specifications, instructions etc.), whether supra or infra, is hereby incorporated by reference in its entirety. Nothing herein is to be construed as an admission that the invention is not entitled to antedate such disclosure by virtue of prior invention. Some of the documents cited herein are characterized as being *"incorporated by reference "*. In the event of a conflict between the definitions or teachings of such incorporated references and definitions or teachings recited in the present specification, the text of the present specification takes precedence.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments, which combine the explicitly described embodiments with any number of the disclosed and/or preferred elements. Furthermore, any permutations and combinations of all described elements in this application should be considered disclosed by the description of the present application unless the context indicates otherwise.

**Definitions**

In the following, some definitions of terms frequently used in this specification are provided. These terms will, in each instance of its use, in the remainder of the specification have the respectively defined meaning and preferred meanings.

As used in this specification and the appended claims, the singular forms "a", "an", and "the" include plural referents, unless the content clearly dictates otherwise.

The term composition used in the present invention refers to a mixture comprising the composition of the first aspect of the present invention and additionally other suitable excipients for topical application to the skin, body and/or scalp. The composition may be in a form of a cream, emulsion, lotion, gel, milk, oil, scrub, powder, mask, or toner or the like. The composition may also be in the form of a foaming cleanser or soap, a shower gel or a bath gel. Furthermore the composition may also be a colour cosmetic product such as a foundation, base for make-up, concealer, pressed powder, mascara or a lipstick. It can also be incorporated into a wrap or film, a mask, a patch, a cloth or blanket, a pad a sheet, a wipe, a pen or the like. The composition may also comprise a vehicle, which may be formulated to improve the delivery of the actives to the skin. The cosmetical and/or topical composition may be formulated in a manner well known in the art of preparing skin care products. The active compounds are generally incorporated in a dermatologically acceptable vehicle or carrier. The composition may be packaged in any suitable manner such as a jar, a bottle, a tube, a pump, a pump dispenser, an aerosol or foam dispensing pump, a roll-back, a stick, a rush or a sachet. It may also be incorporated in a capsule, an ampoule or a pipet.

The delay of sings of aging in the context of the present invention refers to the retardation of the natural process of the skin occurring while lifetime increases. Thus, the delay of signs of aging artificially prolongs the skins intrinsic biological aging mechanisms by adapting to the needs of the skin in a certain age. The signs of aging comprise but are not limited to wrinkles, fine lines, wizened skin, skin laxity associated with collagen loss or destruction, loss or reduction in skin integrity, lack of skin elasticity, lack of skin tone, thinned skin, sagging skin, skin suffering from degradation of collagen fibers, flaccid skin, and skin suffering from internal degradation, for promoting evenness of skin tone by reducing skin redness, blotchiness, for lightening (whitening) the color of the skin or scalp, for enhancing skin desquamation, improving skin hydration, improving skin elasticity, improving skin firmness improving skin luster and brightness, for promoting skin brightness, for promoting skin texture and tone uniformity and/or for reducing the appearance of skin pigmentation and/or skin darkening.

The term plant extract in the context of the present invention refers to a liquid, semisolid or solid preparation, obtained from a plant or parts of a plant or herbal material, which is usually in the dry state. Different types of extracts may be distinguished. Standardised extracts are adjusted within an acceptable tolerance to a given content of constituents with known efficacy of activity. Standardisation is achieved by adjustment of the extract with inert material or by blending batches of extract. Other extracts are essentially defined by their production process (state of raw material to be extracted, solvent, extraction conditions) and their specifications.

Vitamin A like agent in the context of the present invention refers to a compound or a composition, which may be used as an alternative to vitamin A, preferably to retinol. It is preferred that the vitamin A like agent induces no skin irritation. If skin irritation is induced it is preferably less compared to retinol or another vitamin A derivative. The vitamin A like agent has similar effects to retinol but improved tolerance and stability compared to retinol. The vitamin A like agent is not limited in structure to be similar to vitamin A derivatives.

Ubiquinone in the context of the present invention refers to Coenzyme $Q_{10}$, also interchangeably used with ubidecarenone, coenzyme Q, and abbreviated at times to $CoQ_{10}$, CoQ or $Q_{10}$. Ubiquinone is a 1,4-benzoquinone, where Q refers to the quinone chemical group, and 10 refers to the number of isoprene chemical subunits in its tail. This oil-soluble, vitamin-like substance is present in most eukaryotic cells, primarily in the mitochondria. It is a component of the electron-transport-chain participates in cellular respiration, aerobic respiration, generating energy in the form of adenosine triphosphate. Ninety-five percent of the human body's energy is generated this way. Therefore, those organs with the highest energy requirements - such as the heart, liver and kidney have the highest $CoQ_{10}$ concentrations. There are

three states of $CoQ_{10}$: fully oxidized (ubiquinone), semiquinone (ubisemiquinone), and fully reduced (Ubiquinol). The capacity of this molecule to exist in a completely oxidized form and a completely reduced form enables it to perform its functions in the electron transport chain, and as an antioxidant, respectively. The antioxidant nature of $CoQ_{10}$ derives from its energy carrier function. As an energy carrier, the $CoQ_{10}$ molecule continuously goes through an oxidation-reduction cycle. As it accepts electrons, it becomes reduced. As it gives up electrons, it becomes oxidized. In its reduced form, the $CoQ_{10}$ molecule holds electrons rather loosely, so this CoQ molecule will quite easily give up one or both electrons and, thus, act as an antioxidant. $CoQ_{10}$ inhibits lipid peroxidation by preventing the production of lipid peroxyl radicals (LOO). Moreover, $CoQH_2$ reduces the initial perferryl radical and singlet oxygen, with concomitant formation of ubisemiquinone and $H_2O_2$. This quenching of the initiating perferryl radicals, which prevent propagation of lipid peroxidation, protects not only lipids but also proteins from oxidation. In addition, the reduced form of CoQ effectively regenerates vitamin E from the a-tocopheroxyl radical, thereby interfering with the propagation step. Furthermore, during oxidative stress, interaction of $H_2O_2$ with metal ions bound to DNA generates hydroxyl radicals, and CoQ efficiently prevents the oxidation of bases, in particular, in mitochondrial DNA. In contrast to other antioxidants, this compound inhibits both the initiation and the propagation of lipid and protein oxidation. It also regenerates other antioxidants such as vitamin E.

Ectoin as used in the present invention refers to (1,4,5,6-tetrahydro-2-methyl-4-pyrimidinecarboxylic acid) is a natural compound found in several species of bacteria. It is a compatible solute, which serves as a protective substance by acting as an osmolyte and thus helps organisms survive extreme osmotic stress. It stabilizes proteins and other cellular structures and protects the skin from stresses like UV irradiation and dryness. It is preferred that ectoin mediates immune protection and cell protection from damage, protection from UV light, membrane protection and moisture protection with long term effects.

Phytosterols refer to phytosterols, which encompass plant sterols, and are steroid compounds similar to cholesterol, which occur in plants and vary only in carbon side chains and/or presence or absence of a double bond. Stanols are saturated fat sterols, having no double bonds in the sterol ring structure. More than 200 sterols and related compounds have been identified. Free phytosterols extracted from oils are insoluble in water, relatively insoluble in oil, and soluble in alcohols.

Anti-irritant agent describes a diverse group of topical product ingredients, which were able to reduce the irritation potential of other more irritating ingredients in the same product. An anti-irritant is typically an agent, which when used in conjunction with an eye irritant or skin irritants which reduce their irritation potential sufficiently to be tolerated when applied to the body. Three different mechanisms are postulated for the activity shown by various anti-irritants: Some operate by "complexing" the irritant itself. Others react with the skin, blocking reactive sites so that the irritant cannot react with it. A final group seems nonreactive and apparently protects by merely preventing complete physical contacts between irritant and skin An anti-irritant in the context of the present invention can also possess anti-aging properties and is capapble of reducing at least one of the signs of aging as set out below.

The term anti-inflammatory agent refers to a compound or composition which at least is capable of not increasing, diminishing or improving a sign of inflammation. It is preferred that the anti-inflammatory agents leads to an inhibition of inflammatory mediators such as prostaglandins or leukotrienes and thus, results in less typical events of inflammation. Typical signs of inflammation of the skin may comprise redness, heat, pain and swelling of the skin. An anti-irritant in the context of the present invention can also possess anti-aging properties and is capapble of reducing at least one of the signs of aging as set out below.

The term cell growth refers to cell development and cell division (reproduction). When used in the context of cell division, it refers to growth of cell populations, where a cell, known as the "mother cell", grows and divides to produce two "daughter cells" (M phase). When used in the context of cell development, the term refers to increase in cytoplasmic and organelle volume (G1 phase), as well as increase in genetic material (G2 phase) following the replication during S-phase. Cell growth reflects the reproduction of cells, preferably skin cells and enhances renewal of the epidermis and other skin layers. It is preferred that this may result in a decrease of at least one sign if aging of the skin.

Cell metabolism typically reflects the metabolic rate i.e. the conversion of a variety of substances emerging by the different signalling pathways and processes in an eukaryotic cell. Cell metabolism is needed for the degradation of pollutants, contaminants, hazardous substances. Also a normal or increased cell metabolism may be present if the cell is not in a senescent state and shows viability. It is preferred that compounds or compositions of the present invention enhance, improve, increase, positively influence cell metabolism and thus, may result in a delay of at least one sign of skin aging.

Apoptosis refers to the process of programmed cell death. Cellular biochemical events lead to characteristic cell changes (morphology) and in consequence to cell death. These changes include blebbing, cell shrinkage, necrosis and DNA-damage. Two apoptotic pathways are postulated. The TNF-induced) model and the Fas-Fas-mediated model, both involving receptors of the TNF receptor (TNFR) family coupled to extrinsic signals. An antiapoptotic activty of a given compound, substance or composition is characterized in that one or more steps of these signalling pathways, either the intrinsic or the extrinsic pathway are mediated by inhibition or decrease of antiapoptotic signals and thus, result in cell viability and decreased programmed cell death of the respective cells.

The term oxidative stress reflects an imbalance between the systemic manifestation of reactive oxygen species and a biological system's ability to readily detoxify the reactive intermediates or to repair the resulting damage. Disturbances in the normal redox state of cells can cause toxic effects through the production of peroxides and free radicals that damage all components of the cell, including proteins, lipids, and DNA. Further, some reactive oxidative species act as cellular messengers in redox signalling. Thus, oxidative stress can cause disruptions in normal mechanisms of cellular signalling. In humans, oxidative stress is thought to be involved in the development of a variety of diseases i.e. cancer.

A radical scavenger, often interchangeably used with free radical scavenger or antioxidant, refers to a compound that is able to destroy free radicals. A radical scavenger may comprise a vitamin, a mineral or an enzyme. The term free radical refers to a molecule that has one or more unpaired electrons which results in a very unstable molecule. These molecules move through the bloodstream, taking electrons from other cells or giving away unpaired ones. By doing so, free radicals cause cell damage that has been linked to a host of diseases including heart disease and cancer. The role of the free radical scavenger is to hunt down these unstable molecules and destroy them before they can cause significant cell damage within the body. The free radical scavenger is often referred to as an antioxidant. Antioxidants are generally found in certain foods, primarily dark coloured fruits and vegetables e.g. blueberries. These scavengers work by preventing the oxidation process that is required in order for electrons to be passed from one cell to another. The free radical scavenger removes the cells or prevents them from oxidizing simply by being oxidized itself within a close proximity. A body without enough free radical scavengers is at greater risk for housing free radicals and for developing serious diseases later in life.

Dryness of the skin is a very common condition that causes small fine flakes and dry patches. Itching is one of the most common symptoms of dry skin. Given dry skin conditions scratching may be hard to resist. Dry skin is more common in colder winter months and drier climates. While the skin ages elderly people are more prone to dry skin than younger people. Dry skin is more common in individuals with a history of eczema and repeat itch-scratch cycles may lead to skin thickening and darkening. Possible complications include rashes, eczema, and bacterial infections. Extremely dry skin can cause cracks and breaks in the skin. Medications including topical corticosteroids and oral antihistamines can help ease itching. Secondary infections may result from scratches and skin breakdown. Topical or oral antibiotics may be necessary for secondarily infected dry skin. Several home remedies, such as decreasing bathing frequency and lubricating the skin with moisturizers after showers, can help control and prevent dry skin.

Allergy refers to a hypersensitivity disorder of the immune system. Symptoms include red eyes, itchiness, runny nose, eczema, hives, or an asthma attack. Allergies can play a major role in conditions such as asthma. In some people, severe allergies to environmental or dietary allergens or to medication may result in life-threatening reactions called anaphylaxis. Food allergies and reactions to the venom of stinging insects such as wasps and bees are more often associated with these severe reactions. Allergic reactions occur when a person's immune system reacts to normally harmless substances in the environment. A substance that causes a reaction is called an allergen. These reactions are acquired, predictable, and rapid. Allergy is one of four forms of hypersensitivity and is formally called type I (or immediate) hypersensitivity. Allergic reactions are distinctive because of excessive activation of certain white blood cells called mast cells and basophils by a type of antibody called immunoglobulin E (IgE). This reaction results in an inflammatory response which can range from uncomfortable to dangerous. Very common are allergies of the skin after an allergene has been in contact with the skin (so called contact allergy).

Skin hydration refers to a process of providing an adequate amount of liquid to bodily tissues, especially to the skin. With skin hydration it is meant that the amount of hydrating agent or water is applied on the epidermis and then is transported to the deeper skin layers. Such transportation mechanism may comprise absorption, diffusion, active transport, passive transport, osmosis.

The term sign of aging refers to but is not limited to one of the following conditions: the skin may show wrinkles, fine lines, wizened skin, skin laxitiy associated with collagen loss or destruction, loss or reduction in skin integrity, lack of skin elasticity and/or firmness, lack of skin tone, lack of moisture storage capacity of the skin, thinned skin, sagging skin, skin suffering from degradation of collagen fibers, flaccid skin, skin suffering from internal degradation, skin suffering from damage, increased wrinkle depth.

Prevention refers to measures taken for aging prevention, as opposed to aging treatment. Prevention means the trial to avoid, decrease, lower, diminish, decrease the signs of aging while the aging process has not been or only partly initiated. The purpose of prevention is to delay the signs or features of aging skin, especially unwanted features.

The proteasome as referred to in the context of the specification is a proteolytic multicatalytic system responsible for the degradation of oxidized proteins and protein turnover. Through damaged protein removal, the proteasome restores homeostasis in skin cells when disturbed by chronological or photo-induced aging. This enzymatic complex is thus in charge of "housekeeping" cellular cleansing through said degradation of damaged proteins. Proteins are molecules of prime importance for the cells, through the key functions of enzymes. Unfortunately, they are very vulnerable, and the repair system has not been yet as well described as for other components. The proteasome may be found both in cytoplasm and nuclear part of the cell. It presents three enzymatic activities, cleaving specifically different part of the protein. First, harmful oxidized proteins are labeled by a specific molecule composed of 75 amino acids (Ubiquitin). The targeted proteins are then transported to the proteasome, where they are degraded through the three enzymatic activities. Peptides and amino acids are then obtained that may be used again for newly synthesized proteins. The proteasome is down-regulated during replicative senescence as well as in aged cells *in vivo,* possibly resulting in the accumulation of modified proteins. Tests have shown that inhibition of proteasome in young cells induces a senescence-like phenotype, thus demonstrating the fundamental importance of the proteasome for retaining cellular maintenance and homeostasis. It is now well established that proteasome activity decreases during aging. It is dependent of at least three different mechanisms: decreased proteasome expression, alterations and/or replacement of proteasome subunits and formation of cross-linked proteins.

A proteasome activity enhancer as referred to in the context of the present specification is a compound or composition which is capable of stimulating, increasing or enhancing the degradation of oxidized proteins by the proteasome. A proteasome activity enhancer may also protect or repair proteasome activity.

A plant extract from *Cassia angustifolia* refers to an extract derived from the indian plant *Cassia angustifolia.* The extract may be derived from leaves, roots, rhizomas, flowers, blossoms, stipes, cortex or seeds. Such a plant extract of *Cassia angustifolia* may contain natural, botanically derived high molecular weight (HMW) polysaccharides from the seeds of *Cassia angustifolia.* Due to its excellent water binding, film forming and surface smoothing properties, is perfectly suited as botanical alternative to hyaluronic acid.

The term *Phaeodactylum tricornutum* refers to the dominant class of marine phytoplankton, the diatoms, which are microalgae with brown, yellow or green-yellow plasts. One of the most impressive characteristics of diatoms is their ability to form an amorphous silica exoskeleton, named frustule. *Phaeodactylum tricornutum* is a pennate diatom and produces oval, fusiform and triradiate morphs following changes in culture media, light and temperature. The fusiform morphotype is the more common in culture. The size of the cells is about 20-25 $\mu$m length and 4-5 $\mu$m width. The cells are mostly solitary or occasionally connected in pairs. *Phaeodactylum tricornutum* has a wide geographic distribution in coastal marine or brackish waters in temperate zones (Mediterranean, Brittany, Baltic, and Caribbean).

Photoaging refers to the harmful effect of UV radiation on skin, which is related to the generation of reactive oxygen species that alter cellular components including proteins and in turn lead to photodamages of the skin. Sun exposure is one of the major environmental aggression for epidermal cells and responsible for photoaging. Chronic and acute photodamages result from depleted antioxidant enzyme expression and increased oxidative protein modifications. An increase of oxidized proteins is associated with a decrease of proteasome activity in aging epidermal cells as all the three peptidase activities of the proteasome were impaired after UV-A and UV-B irradiation of the cells.

Proteins are composed of amino acids, which may be oxidized, mainly by Reactive Oxygen Species (ROS). They are especially sensitive when containing sulphur (cysteine, methionine) and aromatic amino acids. Oxidized peptides or proteins may lead to inter or intra molecular reticulations and to protein inactivation. With age, oxidized protein accumulation is mainly due to a decrease of protection / repair system activities.

During chronological aging, which refers to the age of a person measured in years, months, and days from the date the person was born, oxidized protein build-up is commonly seen as a hallmark of cellular aging. Several studies have shown the correlation between oxidized protein level and proteasome activity. It is proven that there are increased levels of oxidized proteins, glycated proteins, and proteins modified by the lipid peroxydation products. Further there is a decline of chymotrypsin-like and post-glutamic-like enzyme activity of the proteasome in aging keratinocytes. The proteasome is down-regulated during replicative senescence as well as in aged cells *in vivo,* possibly resulting in the accumulation of modified proteins.

UV-light protectants as referred to within the context of the present specification are compounds or compositions which protect mammalian skin cells from signs of photo aging, skin damages caused by the exposition of skin to UV light. UV-light protectants may comprise sun-creens, UV-filters, organic or inorganic photoprotectant.

Embodiments

In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous. In the work leading to the present invention, it was surprisingly shown that the composition of the present invention has an instantaneously effect on the skin by smoothing and regenerating, increases cell renewal and cell structure and protect the skin from free radicals.

Based on these results the present invention in a first aspect relates to a composition for the delay of at least one sign of skin ageing comprising

a component A comprising:

a) vitamin A like agent

b) ubiquinone, ectoin and/or plant phytosterols;

c) a proteasome activity enhancer;

d) anti-irritant and anti-inflammatory agent and

a component B comprising:

a plant extract from *Cassia angustifolia*.

In a preferred embodiment the vitamin A like agent of component A provides an anti-age effect by increasing the cell growth by mediating cell metabolism and anti-apoptotic activity and acting protective against oxidative stress. Preferably, the vitamin A like agent acts with proven effects similar to retinol: comparable mechanism of action, equivalent performances; but with an improved tolerance and a better stability. Preferably the vitamin A like agent is used for renewing and regenerating skin care preparations and specific care for mature skin. Preferably, the concentration ranges from 5% to 0,01 %, i.e. 4,5%, 4%, 3,5%, 3%, 2,5%, 2%, 1,5%, 1%, 0, 5%, 0,4%, 0,3%, 0,2%, 0,1%, 0,05%, 0,025% or 0,01%. Most preferably the concentration is 0,1%. It is preferred that the vitamin A like agent is derived from *Vigna aconitifolia*. It is even more preferred that the vitamin A like agent is a plant extract from *Vigna aconitifolia*.

In another preferred embodiment of the first aspect of the present invention the component A of the composition comprises ubiquinone and/or ectoin and phytosterols. Preferably, the ubiquinone and/or ectoin and the phytosterols provide cell protection as antioxidants, radical scavengers, by stabilizing biopolymers, UV-light protectants, prevent dryness, allergy and fine dust. In another preferred embodiment of the first aspect of the present invention the ubiquinone in the component A of the composition stabilizes and/or protects cell membranes in skin cells and acts as a radical scavenger and thus, mediates its antioxidizing effect. More preferably the concentration of ubiquinone ranges from 2% to 8 %, i.e. 8, 7,5, 7, 6,5, 6, 5,5, 5, 4,5, 4, 3,5, 3, 2,5, 2%. It is mostly preferred that the ubiquinone ingredient has a concentration of 5%. In another preferred embodiment of the present invention the phytosterols of the composition of the first aspect of the present invention are derived from plants selected from the families of *Dioscoreaceae* or *Fabacea*, more preferably from *Dioscorea villosa* and/or *Glycine max*. Preferably the phytosterols are extracted from *Discorea villosa* and/or *Glycine max*. More preferably, said extracted phytosterols ranges from 2% to 8 %, i.e. 8, 7,5, 7, 6,5, 6, 5,5, 5, 4,5, 4, 3,5, 3, 2,5, 2%. It is mostly preferred that the phytosterol ingredient has a concentration of 5%.

In another embodiment of the first aspect of the present invention the component A of the composition comprises a proteasome activity enhancer. Preferably, the proteasome activity enhancer is derived from *Phaedactylium Tricornutum*, preferably an extract from *Phaedactylium Tricornutum*. It is further preferred that the proteasome activity enhancer prevents chronological aging by stimulating proteasome activities and decreasing oxidized protein rate and also protects and repairs proteasome activities after UV-exposure to limit photoaging. During cellular aging, an accumulation of oxidized proteins is observed. It is mostly preferred that their degradation into peptides and amino acids, and the turnover of amino acids for biosynthesis is mostly ensured by the proteasome which is massively supported in its function by the proteasome activity enhancer. It is also preferred that the extract of *Phaedaetylium Tricornutum* presents a unique composition, very rich in specific fatty acids, more preferably in unsaturated fatty acids. Most preferably the fatty acids of the extract of *Phaedactylium Tricornutum* are selected from the group consisting of oleic acid, linoleic acid, linolenic aicd, palmitoleic acid, docosahexaenoic acid, eicosapentaenoic acid, gadolenic acid, petroselic acid, myristolic aicd, icosenic acid, cetoleinic acid, eruca acid, nervonic acid. Even more preferred is that the fatty is palmitoleic acid, docosahexaenoic acid, eicosapentaenoic acid. It is preferred that these fatty acids act as physiological regulators. It is further preferred that the proteasome activity enhancer acts directly on three proteasome proteolytic activities: LLVY activity, LLE activity, LSTR activity and thus, acts on the oxidized protein content, by reducing it. Further, it helps to maintain and restore the homeostasis of the skin. In another preferred embodiment the component A of the first aspect of the present invention may be incorporated in cosmetic products for activation of the proteasome in order to maintain skin homeostasis and ensure the cell purification, for protection of the proteasome and prevention of its activities impairment upon UV irradiation, to prevents photo-aging, may be used in synergy with filters and anti-oxidants, for repair of proteasome activity after UV irradiation, to prevents the occurrence of aging spots. It is further preferred that the proteasome activity enhancer is comprised in the composition of the first aspect of the present invention in a concentration range from 0,05% to 5%, preferably 0,1%, 0,2%, 0,3%, 0,4%, 0,5%, 0,6%, 0,7%, 0,8%, 0,9%, 1,0%, 1,5%, 2,0%, 2,5%, 3,0%, 3,5%, 4,0%, 4,5%, 5%. More preferably the concentration ranges from 0,1% to 1%, e.g. 0,1%, 0,2%, 0,3%, 0,4%, 0,5%, 0,6%, 0,7%, 0,8%, 0,9%, 1,0%. Most preferably the concentration is 0,3%.

In another preferred embodiment the component A of the composition of the first aspect of the present invention comprises an anti-irritant or anti-inflammatory agent. Preferably this anti-inflammatory or anti-irritant agent is derived from a plant selected from the group consisting of: *Lupunis albus* (*Fabacea*), *Dioscorea* (*Dioscoreaceae*), *Matricaria chamomilla* (*Asteraceae*), *Cardispermum halicacabum* (*Sapindaceae*), *Echium plantagineum* (*Boraginaceae*), *Helianthus* (*Asteracea*) or combinations thereof. Preferably, the anti-inflammatory or anti-irritant agent is derived from roots, leaves, blossoms, flowers, fruits, caulis, rhizomes, and herbs of said plants. More preferably, the anti-inflammatory or anti-irritant agent is a plant extract or a plant oil derived from said plants containing several phytochemicals. It is preferred that this anti-inflammatory or anti-irritant agent is used in a concentration ranging from 0,1% to 10%, e.g. 0,1%, 0,2%, 0,3%, 0,4%, 0,5%, 0,6%, 0,7%, 0,8%, 0,9%, 1%, 1,5%, 2,0%, 2,5%, 3,0%, 3,5%, 4,0%, 4,5%, 5,0%, 6,5%, 7,0%, 7,5%, 8%, 8,5%, 9,0%, 9,5%, 10,0%.

Even more preferably the anti-irritant agent is derived from the *Matricaria chamomilla*. Preferably, the effective anti-irritant agent is bisabolol. It is further preferred that the anti-irritant agent is less irritant and advantageously over azulen, a compound contained in *Matricaria chamomilla* as well. Most preferably bisabolol has a lower allergy rate while exhibiting the same desired anti-irritant effect. Preferably, bisabolol is used in a concentration of 0,1%. It is further preferred that the anti-inflammatory agent decreases signs of inflammation by e.g. inhibiting pathways which result in the production of inflammatory mediators such as prostaglandins or leukotrienes, preferably by the inhibition of key enzymes in the respective pathways. It is further preferred that the anti-inflammatory agent is effective against itching and allergic skin rashes and that it harmonizes and protects irritated skin by protection and repair of the skin barrier. Preferably the anti-inflammatory agent is derived from *Cardiospermum halicacabum.* Even more preferably the anti-inflammatory agent derived from *Cardiospermum halicacabum* comprises phytosterols and/or triterpens. It is also preferred that the anti-inflammatory agent is derived from *Echium plantagineum,* preferably from an oil of *Echium plantagineum.* More preferably, this *Echium plantagineum* derived oil comprises but is not limited to fatty acids, preferably stearidonic acid and is thus capable of inhibiting the key enzyme in leukotriene biosynthesis, the 5-Lipoxygenase. It is also preferred that the anti-inflammatory agent is derived from Helianthus, preferably from an oil of Helianthus. More preferably, this Helianthus derived oil comprises but is not limited to unsaponifiable elements, tocopherol and squalene and thus, resulting in a reduction of lipid peroxidation. Most preferably, the concentration of the anti-inflammatory agent is 0,5%. It is also preferred that the anti-inflammatory agent is derived from Lupunis albus, preferably from an oil of Lupunis albus. In a most preferred embodiment the anti-irritant or anti-inflammatory agent is derived from an oil of Helianthus anuus and is combined with an anti-irritant or anti-inflammatory agent derived from an oil of Lupunis albus.

In another embodiment the composition of the first aspect of the present invention comprises a component B comprising a plant extract from *Cassia angustifolia* and is capable of increasing the degree of moisture/hydration of the skin. It is preferred that a plant extract from Cassia *angustifolia* refers to an extract derived from the indian plant *Cassia angustifolia.* The extract is preferably derived from leaves, roots, rhizomas, flowers, blossoms, stipes, cortex or seeds. Most preferably the extract is derived from seeds of *Cassia angustifolia.* It is also preferred that the plant extract of *Cassia angustifolia* contains natural, botanically derived high molecular weight (HMW) polysaccharides from the seeds of *Cassia angustifolia.* It is further preferred that these HMW polysaccharides form a protective film on the skin that strongly binds water and/or fills up lines and thus, delays at least one sign of aging. It is further preferred that the polysaccharides provide intense moisturization of the skin, which is additionally plumped up, which further adds to reducing fine lines and wrinkles and smoothing of the skin surface. Preferably, the HMW polysaccharides lead to a smoother surface, light reflection and/or increased skin radiance, intense moisturization, reduction of fine lines and/or wrinkles or combinations thereof. Preferably, the composition comprising said plant extract of *Cassia angustifolia* provide a long term, moisturizing care and that such a long term moisturization continues preferably 0,1, 0,2, 0,3, 0,4, 0,5, 0,6, 0,7, 0,8, 0,9, 1,0, 1,5, 2,0, 2,5, 3,0, 3,5, 4,0, 4,5, 5,0, 5,5, 6,0, 6,5, 67,0, 7,5, 8,0, 8,5, 9,0, 9,5, 10,00, 10,5, 11,0, 11,5, 12,00, 12,5, 13,0, 13,5, 14,0, 14,5, 15,0, 15,5, 16,0, 16,5, 17,0, 17,5, 18,0, 18,5, 19,0, 19,5, 20,0, 20,5, 21,00, 21,5, 22,0, 22,5, 23,0, 24,0, 24,5, 25,0, 25,5, 26,0, 26,5, 27,0, 27,5, 28,0, 28,5, 29,0, 29,5, 30,0 hours. Most preferably the long moisturization continues 24 hours. It is preferred that the moisturization/hydration of the skin occurs in the stratum corneum It is preferred that the extract of *Cassia angustifolia* comprising said polysaccharides is used in a concentration ranging from 0,05% to 5%, preferably 0,1%, 0,2%, 0,3%, 0,4%, 0,5%, 0,6%, 0,7%, 0,8%, 0,9%, 1,0%, 1,5%, 2,0%, 2,5%, 3,0%, 3,5%, 4,0%, 4,5%, 5%. More preferably the concentration ranges from 0,1 % to 1%, e.g. 0,1%, 0,2%, 0,3%, 0,4%, 0,5%, 0,6%, 0,7%, 0,8%, 0,9%, 1,0%. Most preferably the concentration is 0,1%.

In another preferred embodiment of the first aspect of the present invention the composition delays at least one sign of aging. It is preferred that the at least one sign of skin ageing is selected from the group consisting of wrinkles, fine lines, wizened skin, skin laxitiy associated with collagen loss or destruction, loss or reduction in skin integrity, lack of skin elasticity, lack of skin tone, lack of moisture storage capacity of the skin, thinned skin, sagging skin, skin suffering from degradation of collagen fibers, flaccid skin, skin suffering from internal degradation, skin suffering from damage, skin suffering from dehydration. Even more preferably, the at least one sign of aging is delayed by the increase of skin hydration, the increase in skin firmness, the increase in skin elasticity or the decreae in wrinkle depth.

In another preferred embodiment of the first aspect of the present invention the composition further comprises excipients suitable for topical application to the skin of the face and/or body and/or scalp; optionally further comprising further cosmetically active agents, moisturizing agents, anti-aging compounds. Further cosmetically active agents comprise but are not limited to retinoids or derivatives thereof, depigmenting agents, sunscreens or UV filters, organic photoprotective agents, peelings agents, inorganic photoprotective agents, antioxidants and mixtures thereof. Excipients suitable for topical application to the skin of the face and/or body and/or scalp are selected from the group consisting of silicones, emulsifiers, thickeners, powders, film formers, delivery enhancers, rheology modifying agents, propellants and combinations thereof. The compositions of the invention may further comprise anti-aging compounds such as vitamins or proteins, skin plumping actives, anti-wrinkle actives, skin purifying actives, peeling agents, sebum reducing agents, mattifying agents, anti-perspirant actives, self-tanning agents. UV filters may comprise ethylhexyl methoxycinnamate, octocrylene, ethylhexyl salic ate, butyl methoxydibenzoylmethane, titanium dioxide or phenylbenzimidazol sulfonic acid, for example.

In a related aspect, the composition of the invention may be provided in the form of a cleansing or protective product for skin, including the face and/or scalp; an anti-wrinkle or anti-ageing composition for the face; a composition for irritated skin; an anti-sun damage composition; an artificial tanning composition for the face and/or body; an after-sun care composition; or an anti-acne/anti-blemish composition. The composition of the invention may be used to delay, treat and/or prevent simultaneously all of the signs of aging, any one of the signs of aging or any combination of two or more of these signs of aging. Suitably, the compositions described herein may be used in any one or all of the anti-aging uses described herein.

In a second aspect the present invention relates to the use of the composition of the first aspect of the present invention for the treatment of and/or prevention of at least one sign of skin ageing, wherein the at least one sign of skin ageing is present on skin of the face, body or scalp of the subject. Preferably the at least one sign of skin ageing is selected from the group consisting of wrinkles, fine lines, wizened skin, skin laxitiy associated with collagen loss or destruction, loss or reduction in skin integrity, lack of skin elasticity, lack of skin tone, lack of moisture storage capacity of the skin, thinned skin, sagging skin, skin suffering from degradation of collagen fibers, flaccid skin, skin suffering from internal degradation, skin suffering from damage. The use also comprises non-therapeutic cosmetic or dermatological procedure to prevent and/or delay and/or limit and/or treat at least one sign of skin ageing or the skin damage condition resulting from intrinsic or photo-induced ageing, comprising the step of applying to the skin, body and/or scalp of a subject an effective amount of the least one compound composition of the invention is envisaged. Desirably, the application of the compounds and or compositions of the invention to the skin, body and/or scalp lightens the color of the skin or scalp of a subject, by preventing the formation and/or removing brown pigment blemishes and/or age blemishes and/or freckles; and/or reduces at least one of skin redness, blotchiness or inflammation; and/or reduces and/or smoothes skin imperfections such as wrinkles and/or fine lines; and/or produces more homogenous, firmer, more toned and more elastic skin; and/or promotes wound healing; and/or puffy eyes and/or dark circles; and/or protects against UV-photo induced damage or degradation of collagen; and/or improves the appearance of acne and/or blemishes. It is preferred that the use of the composition delays at least one sign of aging by promoting evenness of skin tone, by reducing redness, by enhancing skin desquamation, by improving skin hydration, by improving skin luster and brightness, by promoting skin texture. It is also preferred that for the use of the composition of the present invention comprising components A and B the composition is formulated in a cosmetically acceptable mixture for topical application to the skin of the face or body and/ or the scalp.

## Examples

### Example 1: Vit A like PW LS 9898

Water (and) Glycerin (and) *Vigna Aconitifolia* seed extract (and) sodium citrate

Dose of use: 3 to 5%
Aspect: yellow liquid, with a characteristic odor
Solubility: soluble in water and insoluble in fats and oils

### Example 2: Concentration and effects of selected ingredients

| Collageneer | 2,00% | | improving the skin's elasticity remodeling and firming sagging skin areas |
|---|---|---|---|

(continued)

| | | | |
|---|---|---|---|
| **Ectoin** | 2,00% | | preservation of moisture content in the skin: can have a prophylactic effect against dry skin, Anti-Aging reduces signs of aging supports cell protection and thus, protects the immune system of the skin |
| | 0,50% | | moisture increase of the skin |
| | 1,00% | | moisture boost with long-term effect (increases the moisture content in the skin with long-term effect) peservation of the immune system (protects the immune system of the skin) |
| **Hyalurosmooth™** | 3,00% | cream | improves the skin complexion |
| | 10,00% | cream | clearly and significantly improves the overall qualities of skin: softness, suppleness and moisturization |
| **Unisteron Y-50** | 5,00% | o/w cream | improvement of skin hydration improvement of skin lipid content |
| **Vit-A-Like** | 3,00% | cream | stimulates epidermal cellular renewal collagen synthesis from human dermal fibroblasts after treatment of reconstructed epidermis |
| | 5,00% | cream | anti-wrinkle efficacy on the crow's foot area thanks to its cellular renewal activity, Vit-A-LikeTM LS 9793 visibly refines skin texture. stimulation of the epidermis cell renewal and reduction of wrinkles |

**Example 3: Proteasome activity enhancer**

The proteasome activity enhancer is obtained from lipidic extraction of *Phaeodactylum tricornutum.* The marine diatom *Phaeodactylum tricornutum* is widely used in *crustacean* and *mollusc* aquaculture. Its importance is enhanced by other potential applications such as feed supplements. *Phaeodactylum tricornutum* contains a high content of polyunsaturated fatty acid (PUFAs), especially eicosapentaenoic acid. It presents a unique composition, very rich in specific fatty acids: (also refer to Figure 2)

- C16:1
- C20:5, Eicosapentaenoic acid (EPA), an $\omega 3$ polyunsaturated fatty acid
- C22:6, DocosaHexaenoic Acid (DHA), an $\omega 3$ polyunsaturated fatty acid.

**Example 4: Stimulation of proteasome activities *in vitro***

Proteasome activities decrease with age. The test is processed to determine if it is possible to activate the proteasome. Therefore the three proteasome proteolytic activities:

- LLVY-amc (chymotrypsin-like activity)
- LLE-na (post-glutamic-like activity)
- LSTR-amx (trypsin-like activity) are assayed. Selection criteria are as follows:

the test has been processed on Normal Human Keratinocyte lysates. Keratinocytes were treated for 7 hours with 2,5mg/ml of *Phaeodactylum tricornutum* extract. The peptidase activities of the proteasome were assayed in cell lysates using 25 mM of LLVY-AMC, 150 mM of LLE-NA and 40 mM of LSTR-AMC as fluorogenic peptide substrate with 20 mg of total proteins. Proteasome proteolytic activities were determined as the difference between the total activity of cytosolic extracts and the remaining activity in the presence of 20 mM proteasome inhibitor N-Cbz-Leu-Leu-Leucinal (MG132). A statistic test has been calculated. *Phaeodactylum tricornutum* extract activates the three different proteolytic activities of the proteasome. Through this activity, the epidermis is supported to purify from oxidized proteins.

**Example 5: Decrease of oxidized protein level during chronological aging *in vitro***

To assess the effect of *Phaeodactylum tricornutum* extract on oxidized protein level the oxidized proteins with or without treatment with *Phaeodactylum tricornutum* extract were measured. The test has been processed on Normal Human Keratinocytes. Oxyblot (Quantum-Appligene) was performed with 10 $\mu$g of protein from keratinocytes lysates, harvested 7 hours after treatment with 2,5mg/ml of *Phaeodactylum tricornutum* extract. Extracts were first treated with 2,4-DNPH to form the carbonyl derivative dinitrophenyl-hydrazone before SDS/PAGE separation. After electrotransfer, the blots were processed with anti-DNP antibodies. Evaluation was performed by intensitiy of the Oxyblot.

**Example 6: Protection of proteasome activities from UV *in vitro***

Proteasome proteolytic activities are impaired by UV exposure. To assess the effect of *Phaeodactylum tricornutum* extract on these activities the three proteasome peptidase activities after UV irradiation with or without treatment with *Phaeodactylum tricornutum* extract were measured. The test has been processed on Normal Human Keratinocytes. Keratinocytes were treated by 2,5mg/ml of *Phaeodactylum tricornutum* extract for 48 h and irradiated (or not) by 10 J/cm2 of UVA and 0.05 J/cm2 of UVB and then treated again for 7 hours by 2,5mg/ml of *Phaeodactylum tricornutum* extract. Cells are then harvested. The peptidase activities of the proteasome were assayed in cell lysates using 25mM of LLVY-AMC, 150mM of LLE-NA and 40mM of LSTR-AMC as fluorogenic peptide substrate with 20mg of total proteins. Proteasome proteolytic activities were determined as the difference between the total activity of cytosolic extracts and the remaining activity in the presence of 20mM proteasome inhibitor N-Cbz-Leu-Leu- Leucinal (MG132).

**Example 7: Restore of proteasome activities after UV-irradiation**

Proteasome activities are impaired by UV exposure. To assess the effect of *Phaeodactylum tricornutum* extract on these activities after UV irradiation the activities after UV irradiation were measured. The test has been processed on Normal Human Keratinocytes. Keratinocytes were irradiated by 10 J/cm2 of UVA and 0.05 J/cm2 of UVB and/or treated by 2,5mg/ml of *Phaeodactylum tricornutum* extract for 24 hours. Cells are then harvested. The peptidase activities of the proteasome were assayed in cell lysates using 25 mM of LLVY-AMC, 150 mM of LLE- NA and 40 mM of LSTR- AMC as fluorogenic peptide substrate with 20 mg of total proteins. Proteasome proteolytic activities were determined as the difference between the total activity of cytosolic extracts and the remaining activity in the presence of 20 mM proteasome inhibitor N-Cbz-Leu-Leu- Leucinal (MG132). The activities in pmol/min/mg of proteins were evaluated. A student t-test has been evaluated.

**Example 8: Prevention of the increase of UV-induced oxidized proteins *in vitro***

Oxidized proteins appear when the skin is stressed by environmental factors, such as UV exposure. To assess the effect of *Phaeodactylum tricornutum* extract oxidized protein at a normal level, after UV irradiation with or without treatment with *Phaeodactylum tricornutum* extract was measured. The test has been processed on Normal Human Keratinocyte lysates. Oxyblot (Quantum-Appligene) was performed with 10 mg of protein from keratinocytes lysates, harversted 7 hours after treatment with 2,5 ug /ml of *Phaeodactylum* tricornutum extract (cells previously irradiated with 10 J/cm2 of UVA and 0.05 J/cm2 of UVB or not). Extracts were first treated with 2,4-DNPH to form the carbonyl derivative dinitrophenylhydrazone before SDS/PAGE separation. After electrotransfer, the blots were processed with anti-DNP antibodies. The oxyblot intensity was evaluated.

**Example 9: Evaluation of moisturizing activity on stratum corneum of C.angustifolia seed extract**

To evaluate the moisturizing activity on stratum corneum in vitro of 2.5% and 5% of a plant extract from *Cassia angustifolia* in a hydrogel referring to hydrogel with 0.07% and 0.20% sodium (Na) hyaluronate, of the dielectric conductivity was measured. At equivalent concentrations of active matter the extract provided equivalent moisturization as the benchmark, sodium hyaluronate, up to 6 hours. Thus, the seed extract from *Cassia angustifolia* outperformed the benchmark with respect to the long term effect and gave significant 24 hours moisturization.

**Example 10: Production process of the composition of the present invention**

Heat water and add the *Cassia angustifolia* seed extract and let it solve. Add sodium stearyl glutamate and let it solve. Add xanthan and let it solve. Mixing all ingredients of Phase D and homogenize with Phase ABC. Mixing of Phase E, heating to 70°C and adding to the mixture. After that a homogenization step follows. After mixing Phase F, Phase F is added to the mixture. Addition of perfume composition. Slowly agitate till mixture cools down

**Example 11: Cosmetic study desing for the evaluation of skin hydration, biomechanical properties of the skin and wrinkle depth of Intensive Power Serum Line A**

| Number of individuals: | 20 |
|---|---|
| Sex: | Female |
| Age range: | 35-61 (average 48,1) |
| Test Areas: | Inner sides of forearms, Crow's Feet |
| Application: Duration: | 28 days; |
| Frequency: | twice daily |

**Test Parameters:**

1. Determination of skin hydration with Corneometer MPA 5CPU (Courage & Khazaka GmbH, Cologne)
2. Determination of skin firmness and elasticity (biomechanical properties of the skin) by means of Cutometer MPA 580 (Courage & Khazaka GmbH, Cologne)
3. Determination of wrinkle depth by means of PRIMOS® 5.7 high-res (GFMeßtechnik GmbH, Teltow, Germany)

Design of Study ....................... :   **Day 0**

⬛ Determination of the parameters in the test areas

⬛ First test product application
**Day 14**

⬛ Determination of the parameters 8-12 hours following the last respective test product application
**Day 28**

⬛ Determination of the parameters 8-12 hours following the final test product application

Evaluation .............................. :   Descriptive statistics (average, median, minimum, maximum, variance, standard error, standard deviation); Wilcoxon Rank Test

**Performance of Test**

The subjects were selected and were informed about importance and meaning of the study; they could withdraw from the study at any time without giving any reason. Written informed consent was obtained from all the subjects prior to entry into the trial. The following criteria were used for selection of subjects:for inclusion in study:

- female (>18 years of age)

- visible Crow's Feet

- ability to comply with the requirements of the study

- fundamentally clinically healthy

for exclusion from study:

- skin diseases or any other medical condition interfering with the objectives of the study

- planned medical treatment during study period

- pregnancy

A reserve subject in addition to the 20 original subjects, to replace potential drop-outs, started the study with a delay of 1 day (final readings only taken in case a drop-out needed to be replaced). The subjects were instructed not to use any topical preparations on the test areas starting from seven days prior to testing (preconditioning phase) and until the end of the test with exception of the following decorative cosmetics: lipstick/lipliner, mascara/eyeliner and eyeshadow. For cleansing, water or a mild syndet (Eubos® flüssig - blau; manufacturer: Dr. Hobein, D-53340 Meckenheim-Merl, Germany) was allowed only (whole study inclusive the preconditioning phase). Prior to the first application of the test product, measurements were taken at clearly defined sites on the inner sides of the forearms (skin hydration, biomechanical properties - randomized test area assignment) and in the Crow's Feet region (wrinkle depth - randomized side selection). One area on the inner side of the forearms remained untreated and served as control. Further measuring was performed after 14 and 28 days of regular treatment 8-12 hours following the last respective application before the visit. After a demonstration of the correct product application procedure by a Derma Consult staff member, the subjects used the test product (approximately 2 mg/cm$^2$) twice daily (in the morning and evening) at home in a manner corresponding as largely as possible to that to be practised by the future consumer. All measurements were conducted after adaptation to the controlled environmental conditions of the test institute (room temperature: $21\pm1$ °C, relative humidity: $45\pm5\%$).

**Example 12: Measurements of skind hydration (Corneometry)**

The Corneometer MPA 5 CPU (Courage and Khazaka, Cologne, Germany) registers the electrical capacitance of the skin surface. The capacitance is expressed digitally in arbitrary units (a.u.). The probe head (7x7 mm) consisting of a condenser was applied to the skin surface at constant pressure (3.5 N). The measuring principle is based on distinctly different dielectric constants of water (approximately 81) and most other materials (less than seven). Five measurements were performed on each test area and the mean was used to define the hydration state of the stratum corneum.

**Example 13: Measurement of Biomechanical Properties (Elasticity, Firmness)**

The biomechanical properties of the skin are assessed using the Cutometer MPA580 (Courage + Khazaka Electronic GmbH, Cologne; S/N 31050887 tube: S/N 05325678). The measurement is based on the vacuum-suction principle. By applying a constant negative pressure for a given time period, skin is drawn into a hollow tube with an orifice of 2 mm in diameter. Then, at normal air pressure, the skin is allowed to retract. The penetration depth of the skin into the tube is recorded optically without friction and without mechanical influence. A number of standardized parameters can be calculated from the resulting penetration depth curve. Most of the parameters are a function of skin thickness and thus cannot be simply compared between subjects and regions. To increase accuracy and to capture information on the properties of skin under repeated external stress, the cycle is repeated several times and parameters selected for evaluation are based on areas rather than individual measurement points. The delineation of the areas is based on the fittedvlogarithmic envelope curves of the minimum and maximum extensions according to the equation:

$$y \text{⍰} (\ln x \ b)/a$$

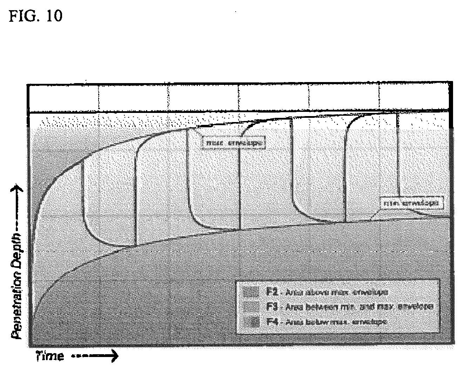

(x = repetitions, y = max. amplitude or min.vamplitude). vThe study was conducted with 20 successive measurement cycles, 1 second suction, 1 second retraction, with a 450 mbar vacuum. The following parameters were selected to assess changes in the biomechanical properties of the skin: (please compare also Figure 10 for cutometer parameters.

**Skin firmness**

Skin firmness is assessed by the parameter F4, the area below the approximated envelope functionof the maximum extensions. ⍰ A decrease in F4 corresponds to an increase in skin firmness.

**Skin elasticity**

Skin elasticity is assessed by the ratio F3 / F4. The larger F3 in comparison to F4, the larger are the restoring forces and the smaller is the remaining residual deformation. ⍰ The closer the resulting value is to 1, the more elastic the skin. The calculation of the parameters was conducted by WinCT (Courage & Khazaka GmbH, Cologne - Germany).

**Example 14: Measurement of wrinkle depth**

PRIMOS (Phase-Shifting rapid in vivo measurement of skin) is a non-contact measurement device that allows for real-time three-dimensional in vivo measurement of the micro topography of human skin based on the technology of active image triangulation. The measurement head consists of a digital micromirror device as projection unit and a CCD-camera as recording unit, mounted onto an adjustable rack. For active image triangulation an intensity encoded point M is projected onto the surface under investigation. Its image on the surface is recorded by the CCD-camera from a specific angle. The point M is a function of parameters like intensity, triangulation angle between projection system and camera and some other inner respectively outer coordinates of the camera and projection plane. The height information of the structured surface is coded in the distorted intensity pattern, which is recorded. The resolution and accuracy depends on the optical and topographical characteristics of the measured surface and on the noise characteristics of the measurement system. For accurate in vivo measurements of human skin, depending on the measured part of the human body (inner forearm, forehead, eye zone), different parameters of effective wavelength and amplification factor should be used. To regard the differences of human skin and avoid undesired distortions by movements, the fast phase-shift technique with settings to accurately detect deeper structures (phase width: 16, 64 & 128 pixels) was used for the measurement. For each measurement, a minimum of 3 recordings were made and the clearest image without movement distortions or artefacts was selected for further processing. On follow-up visits, the original captured data was overlayed to help in the relocation process of the test area. At the end of the study, distortions due to body hairs were digitally removed and the macro structure (calculated by polynomial approximation), i.e. the curvature of the entire test area, subtracted to allow a proper analysis of the microstructure, i.e. wrinkles and surface roughness. Wrinkle depth was then assessed by means of the parameter RMax that is defined as the maximum vertical distance from the highest peak to the lowest valley of five segments of equal length. To mitigate locational effects, the evaluation was conducted using the arithmetic average of Rmax from 50 parallel cuts. System used in this study: PRIMOS compact high-res S/N 108-00041, Software Version 5.7.

**Claims**

1. A composition for the delay and/or improvement of at least one sign of skin ageing comprising

   a component A comprising:

       a) a vitamin A like agent;
       b) ubiquinone, ectoin and/or plant phytosterols;
       c) a proteasome activity enhancer;
       d) an anti-irritant and anti-inflammatory agent and

   a component B comprising:

       a plant extract from *Cassia angustifolia*.

2. The composition according to claim 1 wherein the vitamin A like agent of component A provides an anti-age effect by increasing the cell growth by mediating cell metabolism and anti-apoptotic activity and acting protective against oxidative stress.

3. The composition according to claims 1 to 2 wherein the Vitamin A like agent is derived from a plant extract from *Vigna aconitifolia*.

4. The composition according to claims 1 and 3 wherein ubiquinone and/or ectoin and phytosterols of component A provide cell protection as antioxidants, radical scavengers, by stabilizing biopolymers, UV-light protectants, prevent dryness, allergy and fine dust.

5. The compositing according to claims 1 to 4 wherein comprises the phytosterols of component A are selected from *Dioscoraceae* and/or *Fabaceae*.

6. The composition according to claims 1 to 5 wherein the anti-irritant or anti-inflammatory agent is selected from the group consisting of the following plants: *Lupunis albus* (*Fabacea*), *Dioscorea* (*Dioscoreaceae*), *Matricaria chamomilla* (*Asteraceae*), *Cardispermum halicacabum* (*Sapindaceae*), *Echium plantagineum* (*Boraginaceae*), *Helianthus* (*Asteracea)*.

7. The composition according to claims 1 to 6 wherein the plant extract of *Cassia angustifolia* of component B improves skin hydration and/or hydration storage..

8. The composition according to claim 7 wherein the plant extract of *Cassia angustifolia* contains natural, botanically derived high molecular weight polysaccharide from the seeds of *Cassia angustifolia.*

9. The composition according to claim 8 wherein the polysaccharides provide moisturization/hydration of the skin.

10. The composition according to claims 8 to 9 wherein the polysaccharides lead to a smoother skin surface, higher light reflection and/or increased skin radiance.

11. The composition according to claims 1 to 10 wherein the at least one sign of skin ageing is selected from the group consisting of wrinkles, increase in wrinkle depth, fine lines, wizened skin, skin laxitiy associated with collagen loss or destruction, loss or reduction in skin integrity, lack of skin elasticity, lack of skin firmness, lack of skin tone, lack of moisture storage capacity of the skin, thinned skin, sagging skin, skin suffering from degradation of collagen fibres, flaccid skin, skin suffering from internal degradation, skin suffering from damage.

12. The composition according to claims 1 to 11 comprising further excipients suitable for topical application to the skin of the face and/or body and/or scalp; optionally further comprising further cosmetic agents, moisturizing agents, anti-aging compounds and combinations thereof.

13. Use of a composition according to claims 1 to 12 for the treatment of and/or prevention of at least one sign of skin ageing, wherein the at least one sign of skin ageing is present on skin of the face, body or scalp of the subject.

14. Use according to claim 13 for promoting evenness of skin tone by reducing redness, for enhancing skin desquamation, for improving skin hydration, skin firmness, skin elasticity, for decreasing wrinkle depth of the skin, for improving skin luster and brightness, for promoting skin texture and combinations thereof.

15. Use according to claims 13 to 14 wherein the composition comprising components A and B is formulated in a cosmetically acceptable mixture for topical application to the skin of the face or body and/ or the scalp.

FIG.1

**D0**

**D56**

FIG.2

FIG.3

Stimulation effect on proteasome
LLVY activity

Stimulation effect on proteasome
LSTR activity

Stimulation effect on proteasome
LLE activity

\* UV vs control

FIG. 4

EP 3 090 781 A1

FIG. 5

Protection effect on proteasome LLVY activity upon UV irradiation

Protection effect on proteasome LSTR activity upon UV irradiation

Protection effect on proteasome LLE activity upon UV irradiation

* UV vs control
* Phaeo vs UV

FIG. 6

FIG. 7

FIG. 8

% increase of dielectric conductivity (active product *versus* placebo)

Hydrogels containing:

▨ 0.07% Na hyaluronate
▨ 0.20% Na hyaluronate

▨ 2.5% Hyalurosmooth™*
▨ 5% Hyalurosmooth™*

Statistics:
Percentage calculated
on the basis of the mean
from 10 experiments
Mann-Whitney's U test
(*) Significant / control
(**) Significant / control
and placebo

2.5% and 5% Hyalurosmooth™* = 0.07% and 0.20% hyaluronic acid in terms of active matter

FIG. 9

| Ingredient: | | amount [g] | | |
|---|---|---|---|---|
| **Phase A** | | | | |
| 1 | Wasser | Metzler | | 54,75 |
| 2 | Hyalurosmooth PW LS 8997 | Impag | | 0,10 |
| **Phase B** | | | | |
| 3 | Eumulgin SG | BTC Europe | | 1,00 |
| **Phase C** | | | | |
| 4 | Keltrol CG-SFT | Rahn | | 0,20 |
| **Phase D** | | | | |
| 5 | Glycerin 99% pflanz. | Brenntag | | 3,00 |
| 6 | Dermosoft OMP | Dr. Streatmanns | | 4,00 |
| 7 | Pentavitin | Plantapharm | | 1,00 |
| **Phase E** | | | | |
| 8 | Tego Alkanol 1618 | Franken Chemie | | 2,00 |
| 9 | Dermofeel GSC | Dr. Streatmanns | | 2,00 |
| 10 | Squalan | Henry Lamotte | | 8,00 |
| 11 | Cetiol RLF | BTC Europe | | 8,00 |
| 12 | Defensil | Rahn | | 0,50 |
| 13 | Unisteron Y-50 | Plantapharm | | 5,00 |
| 14 | Dermofeel Toco 70 non-GMO | Dr. Streatmanns | | 0,20 |
| **Phase F** | | | | |
| 15 | Bisabolol Alpha | Pointner % Roth. Selco | | 0,10 |
| 16 | Zitronensäure 30% | Akras | | 0,10 |
| 17 | Vit.A-Like PW LS 9898 | Impag | | 0,25 |
| 18 | Collageneer | Seppl (Expansience) | | 2,00 |
| 19 | Ectoin | Merk | | 2,00 |
| 20 | Megassane | Impag | | 0,30 |
| 21 | Rovisome Q10 | Rovi /Air product | | 5,00 |
| **Phase G** | | | | |
| 22 | Waterlily | Cosnaderm | | 0,50 |
| **Total** | | | | 100,00 |

FIG. 10

FIG. 11a

Experimental data of Skin Hydration (delta values)

FIG. 11b

Experimental data of Skin Hydration

Fig. 11c

Increase in Skin Hydration relative to initial conditions and to untreated

Fig. 12a

Fig. 12b

EP 3 090 781 A1

## Experimental data of Skin Firmness

DCC16W020

Mean Cutometer readings (f4)

| | start | after 14 days | after 28 days |
|---|---|---|---|
| untreated | 18.99 | 19.32 | 19.23 |
| Intensiv Wirkstoffserum Linie A / Intensive Power Serum Line A | 18.90 | 17.63 | 16.57 |

Increase in Skin Firmness relative to initial conditions and to untreated

Fig. 12c

EP 3 090 781 A1

33

Fig. 13a

Fig. 13b

Experimental data of Skin Elasticity

| | start | after 14 days | after 28 days |
|---|---|---|---|
| ■ untreated | 0.68 | 0.67 | 0.67 |
| ☐ Intensiv Wirkstoffserum Linie A / Intensive Power Serum Line A | 0.67 | 0.70 | 0.73 |

DCC16W020

Mean Cutometer readings (r7/r4)

Fig. 13c

Fig. 14a

EP 3 090 781 A1

37

## Experimental data of Wrinkle Depth (delta values)

DCC16W020

Mean PRIMOS readings (Rmax, µm) - to baseline

| | after 14 days | after 28 days |
|---|---|---|
| ⊞ Intensiv Wirkstoffserum Linie A / Intensive Power Serum Line A | -35.23 | -62.92 |

*p<0.05 versus initial condition

Fig. 9: ΔRmax Values

## Experimental data of Wrinkle Depth

DCC16W020

Mean PRIMOS readings (Rmax, µm)

| | start | after 14 days | after 28 days |
|---|---|---|---|
| Intensiv Wirkstoffserum Linie A / Intensive Power Serum Line A | 417.45 | 382.22 | 354.53 |

Fig. 14b

Fig. 14c

Decrease in Wrinkle Depth relative to initial conditions

Europäisches Patentamt

European Patent Office

Office européen des brevets

# EUROPEAN SEARCH REPORT

Application Number

EP 16 00 1045

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | "The Essentials by Beauty Creations - Cosmetic actives for skin & hair care", , 11 September 2012 (2012-09-11), XP055228310, Retrieved from the Internet: URL:http://www.in-cosmeticsasia.com/__nova documents/17301?v=634859782296070000 [retrieved on 2015-11-13] * pages 5,7,9 * * pages 13,21 * | 1-15 | INV. A61Q19/08 A61K8/35 A61K8/63 A61K8/49 A61K8/97 A61K8/99 |
| Y | "Unisteron Y-50", , 1 January 2009 (2009-01-01), XP055228265, Retrieved from the Internet: URL:http://www.induchem.com/wp-content/fil es_mf/1361792973Publication_Unisteron_Y50. pdf [retrieved on 2015-11-13] * the whole document * | 1-15 | |
| Y | "Collageneer", , 1 March 2014 (2014-03-01), XP055228269, Retrieved from the Internet: URL:https://www.in-cosmeticskorea.com/__no vadocuments/87221?v=635678010711530000 [retrieved on 2015-11-13] * the whole document * | 1-15 | TECHNICAL FIELDS SEARCHED (IPC) A61Q A61K |

-/--

The present search report has been drawn up for all claims

| Place of search | Date of completion of the search | Examiner |
|---|---|---|
| Munich | 28 June 2016 | Giacobbe, Simone |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 16 00 1045

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Anonymous: "Total Age Defy Cream", , 1 March 2015 (2015-03-01), XP055228155, Retrieved from the Internet: URL:http://www.gnpd.com/sinatra/recordpage /3006877/from_search/FhzJz8Rey0/ [retrieved on 2015-11-12] * the whole document * | 1-15 | |
| Y | Anonymous: "GNPD - Vitamin A Plus Serum", , 1 April 2012 (2012-04-01), XP055228190, Retrieved from the Internet: URL:http://www.gnpd.com/sinatra/recordpage /1777224/from_search/K8W80mzPf4/ [retrieved on 2015-11-12] * the whole document * | 1-15 | |
| Y | Anonymous: "GNPD - Esting Beauty Lotion I", , 1 February 2015 (2015-02-01), XP055228261, Retrieved from the Internet: URL:http://www.gnpd.com/sinatra/recordpage /2999121/from_search/EvBYRQFJCi/ [retrieved on 2015-11-13] * the whole document * | 1-15 | TECHNICAL FIELDS SEARCHED (IPC) |
| Y | Anonymous: "GNPD - Omega 3 Optimum Skin Serum Oil", , 1 October 2013 (2013-10-01), XP055228274, Retrieved from the Internet: URL:http://www.gnpd.com/sinatra/recordpage /2213950/from_search/wSVwgovMIB/ [retrieved on 2015-11-13] * the whole document * | 1-15 | |

-/--

The present search report has been drawn up for all claims

| Place of search | Date of completion of the search | Examiner |
|---|---|---|
| Munich | 28 June 2016 | Giacobbe, Simone |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.............................................................
& : member of the same patent family, corresponding document

EPO FORM 1503 03.82 (P04C01)

**Europäisches Patentamt**
**European Patent Office**
**Office européen des brevets**

# EUROPEAN SEARCH REPORT

Application Number

EP 16 00 1045

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Anonymous: "GNPD - Lift & Moisturize Daily Cream", , 1 October 2010 (2010-10-01), XP055228235, Retrieved from the Internet: URL:http://www.gnpd.com/sinatra/recordpage /1405519/from_search/pDXp9nVuN1/ [retrieved on 2015-11-13] * the whole document * | 1-15 | |
| Y | US 2004/136945 A1 (NIZARD CARINE [FR] ET AL) 15 July 2004 (2004-07-15) * the whole document * | 1-15 | |
| Y | US 2010/074879 A1 (AOKI HIROBUMI [JP] ET AL) 25 March 2010 (2010-03-25) * the whole document * | 1-15 | |
| Y | US 2004/132699 A1 (ZHUANG JOHN CHENGFENG [US] ET AL) 8 July 2004 (2004-07-08) * the whole document * | 1-15 | |
| Y | US 2001/021388 A1 (MOTITSCHKE LOTHAR [DE] ET AL) 13 September 2001 (2001-09-13) * the whole document * | 1-15 | TECHNICAL FIELDS SEARCHED (IPC) |

The present search report has been drawn up for all claims

| Place of search | Date of completion of the search | Examiner |
|---|---|---|
| Munich | 28 June 2016 | Giacobbe, Simone |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 00 1045

This annex lists the patent family members relating to the patent documents cited in the above-mentioned European search report.
The members are as contained in the European Patent Office EDP file on
The European Patent Office is in no way liable for these particulars which are merely given for the purpose of information.

28-06-2016

| Patent document cited in search report | | | Publication date | Patent family member(s) | | | Publication date |
|---|---|---|---|---|---|---|---|
| US 2004136945 | A1 | | 15-07-2004 | AT | 520391 | T | 15-09-2011 |
| | | | | AU | 2002251159 | A1 | 21-10-2002 |
| | | | | CN | 1499957 | A | 26-05-2004 |
| | | | | EP | 1372598 | A2 | 02-01-2004 |
| | | | | FR | 2822701 | A1 | 04-10-2002 |
| | | | | HK | 1063291 | A1 | 26-05-2006 |
| | | | | JP | 4180924 | B2 | 12-11-2008 |
| | | | | JP | 2004525160 | A | 19-08-2004 |
| | | | | KR | 20030089709 | A | 22-11-2003 |
| | | | | US | 2004136945 | A1 | 15-07-2004 |
| | | | | WO | 02080876 | A2 | 17-10-2002 |
| US 2010074879 | A1 | | 25-03-2010 | CN | 101522161 | A | 02-09-2009 |
| | | | | EP | 2081544 | A1 | 29-07-2009 |
| | | | | JP | 5180556 | B2 | 10-04-2013 |
| | | | | JP | 2008115170 | A | 22-05-2008 |
| | | | | KR | 20090066324 | A | 23-06-2009 |
| | | | | US | 2010074879 | A1 | 25-03-2010 |
| | | | | WO | 2008047882 | A1 | 24-04-2008 |
| US 2004132699 | A1 | | 08-07-2004 | AU | 771934 | B2 | 08-04-2004 |
| | | | | AU | 4699200 | A | 05-12-2000 |
| | | | | BR | 0010619 | A | 19-02-2002 |
| | | | | CA | 2373158 | A1 | 23-11-2000 |
| | | | | CN | 1351487 | A | 29-05-2002 |
| | | | | CZ | 20014004 | A3 | 15-05-2002 |
| | | | | EP | 1181006 | A1 | 27-02-2002 |
| | | | | JP | 2002544217 | A | 24-12-2002 |
| | | | | KR | 100455470 | B1 | 06-11-2004 |
| | | | | MX | PA01011801 | A | 24-04-2002 |
| | | | | US | 2002098218 | A1 | 25-07-2002 |
| | | | | US | 2004132699 | A1 | 08-07-2004 |
| | | | | WO | 0069404 | A1 | 23-11-2000 |
| US 2001021388 | A1 | | 13-09-2001 | AT | 252366 | T | 15-11-2003 |
| | | | | DE | 4342560 | A1 | 22-06-1995 |
| | | | | DK | 0671161 | T3 | 09-02-2004 |
| | | | | EP | 0671161 | A1 | 13-09-1995 |
| | | | | ES | 2208647 | T3 | 16-06-2004 |
| | | | | FI | 945838 | A | 15-06-1995 |
| | | | | JP | 3635703 | B2 | 06-04-2005 |
| | | | | JP | H07330535 | A | 19-12-1995 |
| | | | | PT | 671161 | E | 31-03-2004 |
| | | | | US | 6060071 | A | 09-05-2000 |
| | | | | US | 6267973 | B1 | 31-07-2001 |
| | | | | US | 2001021388 | A1 | 13-09-2001 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 16 00 1045

This annex lists the patent family members relating to the patent documents cited in the above-mentioned European search report.
The members are as contained in the European Patent Office EDP file on
The European Patent Office is in no way liable for these particulars which are merely given for the purpose of information.

28-06-2016

| Patent document cited in search report | Publication date | Patent family member(s) | Publication date |
|---|---|---|---|
| | | | |

For more details about this annex : see Official Journal of the European Patent Office, No. 12/82